# EUROPÄISCHE PATENTSCHRIFT

(11) **EP 1 410 002 B1**
(45) Veröffentlichungstag und Bekanntmachung des Hinweises auf die Patenterteilung: **24.06.2009**
(21) Anmeldenummer: 02740188.4
(22) Anmeldetag: 04.07.2002
(51) Int. Cl.: G01N 21/89, D06H 3/08, G01N 33/36

(54) **VERFAHREN ZUR ERKENNUNG VON FREMDSTOFFEN IN EINEM TEXTILEN MATERIAL**
METHOD FOR IDENTIFYING FOREIGN BODIES IN A TEXTILE MATERIAL
PROCEDE POUR IDENTIFIER DES CORPS ETRANGERS DANS UN MATERIAU TEXTILE

(30) Priorität: 12.07.2001 CH 128101
(43) Veröffentlichungstag der Anmeldung: 21.04.2004
(73) Patentinhaber: Uster Technologies AG, 8610 Uster (CH)
(72) Erfinder: PIRANI, Peter, CH-8624 Grüt/Gossau (CH); WAMPFLER, Hans, CH-8049 Zürich (CH)
(86) Internationale Anmeldenummer: PCT/CH2002/000364
(87) Internationale Veröffentlichungsnummer: WO 2003/008950

(56) Entgegenhaltungen:
- EP-A- 0 652 432
- EP-A- 1 058 112
- WO-A-00/73771
- WO-A-95/29396
- DE-U- 29 719 245
- US-B1- 6 201 602

## Beschreibung

Die Erfindung betrifft ein Verfahren zur Erkennung von Fremdstoffen in einem textilen Grundmaterial, wobei das textile Grundmaterial mit Strahlung beaufschlagt wird und die am Grundmaterial reflektierte Strahlung erfasst und in ein elektrisches Signal gewandelt wird.

Als textiles Grundmaterial verstehen wir hier beispielsweise einen Faserverbund aus textilen Fasern wie beispielsweise ein Band, ein Vorgarn, ein Garn, beispielsweise aus Baumwoll- oder Polyesterfasern oder aus Mischungen davon usw., oder einen Verbund aus einem solchen Faserverbund wie beispielsweise ein Vlies, ein Gewebe, eine Gewirke usw. Als textiles Grundmaterial verstehen wir ein Grundmaterial, das Fremdstoffe enthalten kann, aber mengenmässig vorliegt. Solche Fremdstoffe sind beispielsweise Fremdfasern im Faserverbund, Teile von Plastikfolien, die beispielsweise zu Bändchen oder Fasern zerkleinert sind, Vegetabilien (wie z.B. Schalenteile) der Baumwolle und sonstige unerwünschte Stoffe wie beispielsweise Haare, Federn usw., die als Bestandteile aus Pflanzen auftreten.

Aus der WO 95/29396 ist ein solches Verfahren und eine Vorrichtung bekannt. Dabei wird das Grundmaterial, hier beispielsweise ein Garn, vor einem absorbierenden Hintergrund mit weissem Licht aus einer Lichtquelle oder abwechslungsweise mit Licht verschiedener Wellenlängen, also beispielsweise gelbem, grünem und rotem Licht aus verschiedenen Lichtquellen bestrahlt Das am Grundmaterial reflektierte Licht wird von mindestens einem Empfänger erfasst, der spektral unterschiedliche Anteile der Strahlung zeitlich getrennt erfasst. So kann ein einziger Empfänger abwechslungsweise die verschiedenen Farben empfangen, oder für jede Farbe ist ein eigener Empfänger vogesehen, so dass alle Farben gleichzeitig durch verschiedene Empfänger erfasst werden. Der oder die Empfänger erzeugen je ein elektrisches Signal, das der bevorzugt empfangenen Wellenlänge der Strahlung entspricht. Diese Signale werden anschliessend zueinander in Beziehung gebracht, beispielsweise miteinander verrechnet, um unerwünschte Einflüsse auszugleichen. Ein solcher unerwünschter Einfluss liegt beispielsweise darin, dass das reflektierte Licht nicht nur von der Farbe des Garns und des Fremdstoffes, sondern auch von der Masse, dem Volumen oder vom Durchmesser des Garns abhängt. Durch die genannte Verrechnung der Signale kann beispielsweise der Einfluss der Masse oder des Volumens ausgesondert werden, so dass beispielsweise nur der Einfluss der Farbe des Garns erkennbar wird. So kann ein Fremdstoff im Material sicher erkannt werden.

Ein Nachteil dieses Verfahrens oder der Vorrichtung ist darin zu sehen, dass die Verarbeitung der Signale aufwändig ist, da die Signale der einzelnen Farb- oder Wellenlängenanteile entweder zeitlich durch mehrere getaktete Lichtquellen oder örtlich durch Filter getrennt werden müssen. Dann werden die einzelnen Signale auch getrennt verarbeitet und später zusammen verrechnet oder gemeinsam weiterverarbeitet. Das bekannte Verfahren erlaubt es aber nicht, bei mehreren verschiedenen Fremdstoffen, die im Grundmaterial örtlich verteilt vorhanden sind, die einzelnen Fremdstoffe getrennt zu erfassen, sodass zwischen den verschiedenen Fremdstoffen differenziert werden kann, um beispielsweise einzelne Fremdstoffe aus dem Grundmaterial auszuscheiden und andere bewusst im Grundmaterial zu belassen.

Die US-6,201,602 B1 offenbart ein Verfahren zum Erfassen von Fremdmaterialien in einem Textilmaterial. Das Textilmaterial wird in ein Messvolumen eingebracht. Es wird mit Messlicht aus einer Lichtquelle beleuchtet, wobei Fremdmaterial und Textilmaterial das Messlicht unterschiedlich absorbieren. Messlicht aus dem gesamten Messvolumen wird mit mindestens einem Photodetektor erfasst. Der Photodetektor erzeugt ein erstes elektrisches Signal, wenn das Textilmaterial im Messvolumen vorhanden ist, und ein zweites elektrisches Signal, wenn kein oder nicht verunreinigtes Textilmaterial im Messvolumen vorhanden ist. Durch Vergleichen der beiden Signale wird ein Absorptionswert aus dem ersten Signal abgeleitet. Aus dem Absorptionswert wird in einer Signalverarbeitungseinheit bestimmt, ob Fremdmaterial im Textilmaterial vorhanden ist.

Es ist deshalb die Aufgabe der Erfindung, die genannten Nachteile zu vermeiden und ein Verfahren vorzuschlagen, das es erlaubt, gezielt verschiedene Fremdstoffe im Grundmaterial so zu erfassen, dass sie auch voneinander unterschieden werden können, so dass alle oder nur die unerwünschten Fremdstoffe erkannt und z.B. ausgeschieden werden können. Es soll damit insbesondere dann diese Möglichkeit geboten werden, wenn vorausgehend ein Verdacht besteht, dass zwei oder mehrere unterschiedliche Fremdstoff-Typen vorhanden sein könnten.

Zur Lösung dieser Aufgabe geht man davon aus, dass das Ausmass der Reflexion von Strahlen am textilen Grundmaterial und an möglichen Fremdstoffen mindestens teilweise bekannt ist, und dass Unterschiede im Ausmass der Reflexion bestehen, die sich in Funktion der Wellenlänge der angewendeten Strahlen ergeben. Während zum Beispiel Baumwolle als Grundmaterial einen stetigen Verlauf der Reflexion in Funktion von Veränderungen der Wellenlänge der Strahlen aufweist, so trifft das nicht für alle möglichen Fremdstoffe zu. Es kann sein, dass gewisse Fremdstoffe einen fast Willkürlich anmutenden Verlauf aufweisen und bei gewissen Wellenlängen Strahlen gleich stark reflektieren wie das Grundmaterial, aber bei anderen Wellenlängen weit weniger oder weit mehr. So können auch die Fremdstoffe unter sich je nach Wellenlänge der Strahlen teils gleich, teils unterschiedlich stark reflektieren.

Es ist vorteilhaft, sich zuerst darüber Kenntnis zu verschaffen, wie das Grundmaterial und wie die gesuchten Fremdstoffe in gewählten Wellenlängenbereichen Strahlen reflektieren.

Entscheidend ist, dass die ausgesendeten Strahlen im Wesentlichen zwei begrenzte und unterschiedliche Wellenlängenbereiche aufweisen und dass die reflektierten Strahlen für beide Wellenlängenbereiche gleichzeitig und gemeinsam erfasst werden. Als begrenzten Wellenlängenbereich verstehen wir einen solchen mit bestimmter spektraler Verteilung um eine zentrale Wellenlänge. Der begrenzte Wellenlängenbereich ist schmalbandig.

Ein erster begrenzter Wellenlängenbereich der Strahlen soll gezielt so gewählt werden, dass die am Grundmaterial reflektierten Strahlen für mindestens zwei verschiedene Fremdstoffe im elektrischen Signal erste Werte ergeben, die beliebig sein können. Dann soll für die Strahlen ein zweiter begrenzter Wellenlängenbereich so gewählt werden, dass die reflektierten Strahlen für die Fremdstoffe im elektrischen Signal zweite Werte ergeben, die für die beiden Fremdstoffe nicht im gleichen Verhältnis stehen wie bei den entsprechenden elektrischen Signalen im ersten Wellenlängenbereich. Die Leistung der Strahlen in den beiden Wellenlängenbereichen soll so gewählt werden, dass die reflektierte Strahlung für die beiden Fremdstoffe unterschiedliche Werte ergibt.

Dies kann mit einer oder mit mehreren Strahlungsquellen erreicht werden, die Strahlen in diesen beiden Wellenlängenbereichen auf das Grundmaterial richten, in denen hinsichtlich Intensität der Reflexion für die beiden Fremdstoffe genügende Unterschiede erwartet werden können. Ein einziger Empfänger dient der Messung der am Grundmaterial insgesamt reflektierten Strahlen.

Vorzugsweise sollen die Strahlen, sofern sie aus verschiedenen Strahlungsquellen stammen, gemischt werden, so dass ein homogenes Strahlengemisch auf das Grundmaterial auftrifft. Die davon abgestrahlte Gesamtstrahlung ist dann proportional zur Summe der Beiträge der Reflexionen in den einzelnen Wellenlängenbereichen. Diese Gesamtstrahlung kann somit in einem einzigen Sensor gleichzeitig erfasst werden. Dieser gibt auch ein Signal ab, das unmittelbar verwendbar ist, beispielsweise um eine Trennvorrichtung zu betätigen, mit der jener Teil des Grundmaterials entfernt werden kann, der unerwünschte Fremdstoffe enthält.

Die durch die Erfindung erreichten Vorteile sind insbesondere darin zu sehen, dass auf diese Weise in einem textilen Grundmaterial gezielt nach bestimmten Fremdstoffen gesucht werden kann. Es wird damit möglich zu wählen, welche Fremdstoffe gezielt beseitigt und weiche allenfalls im Grundmaterial belassen werden können. Sind getrennte und steuerbare Strahlungsquellen vorgesehen, so kann die Vorrichtung die Arbeitsweise beispielsweise an andere Fremdstoffe anpassen, die ursprünglich vorhandene Fremdstoffe plötzlich oder nach und nach im selben Grundmaterial ablösen oder ergänzen, indem die Intensität der Strahlen mit den beiden Wellenlängenbereichen an die sich ändernden Verhältnisse angepasst werden. So lässt sich die Vorrichtung auch an die Bedürfnisse der Betreiber oder Kunden laufend anpassen. Beispielsweise kann damit bestimmt werden, ob vegetabile Fremdstoffe ausgereinigt oder eben im Grundmaterial belassen werden sollen. Wenn es durch die gewählte Auslegung der Vorrichtung möglich ist, die relative Intensität der Strahlen in den beiden Wellenlängenbereichen anzupassen, so kann man durch Anpassung der Empfindlichkeit des angeschlossenen Reinigers erreichen, dass auch der Ausreinigungsgrad der vegetabilen Fremdstoffe gesteuert werden kann.

Im folgenden wird die Erfindung anhand eines Beispiels und mit Bezug auf die beiliegenden Zeichnungen näher erläutert. Es zeigen:
Fig. 1, 2 und 3 je eine Darstellung eines Fremdstoffes in einem Grundmaterial,
Fig. 4 eine vereinfachte Darstellung von Reflexionen am Grundmaterial und an Fremdstoffen durch Strahlen in verschiedenen Wellenlängenbereichen,
Fig. 5 eine schematische und kombinierte Darstellung der erfindungsgemäss erzeugten Signale für das Grundmaterial und für verschiedene Fremdstoffe,
Fig: 6 eine schematische Darstellung einer offenbanten Vorrichtung und
Fig. 7, 8, 9 und 10 je eine schematische Darstellung eines Teils der Vorrichtung.

Das erfindungsgemässe Verfahren soll anschliessend an einem Beispiel erläutert werden, bei dem das Grundmaterial durch ein Garn gebildet ist.

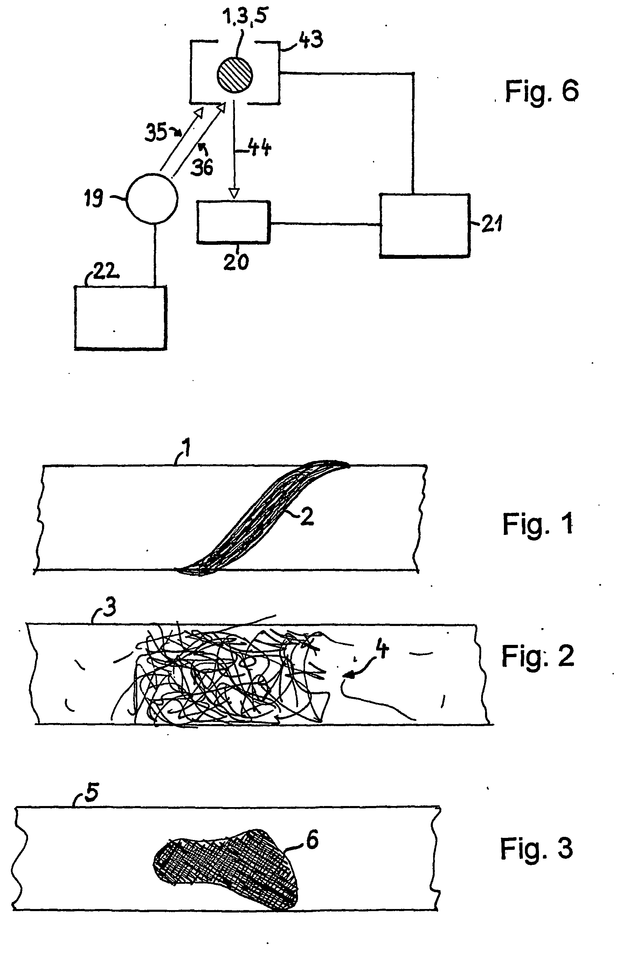

Fig. 1 zeigt ein Grundmaterial oder Garn 1, das als Fremdstoff 2 ein Stück einer Folie aufweist, die das Garn spiralförmig umwickelt.

Fig. 2 zeigt ein Grundmaterial als Band, Vlies oder Garn 3, das als Fremdstoff 4 eine Ansammlung von Einzelfasern aufweist, die eine andere Farbe als die Farbe des Grundmaterials aufweisen.

Fig. 3 zeigt ein Grundmaterial als Band, Vlies oder Garn 5, das als Fremdstoff 6 einen Einschluss oder ein kompaktes Gebilde aufweist, wie z.B. ein beliebiger Fremdkörper, ein Schalenteil der Baumwollpflanze, eine Nisse usw.

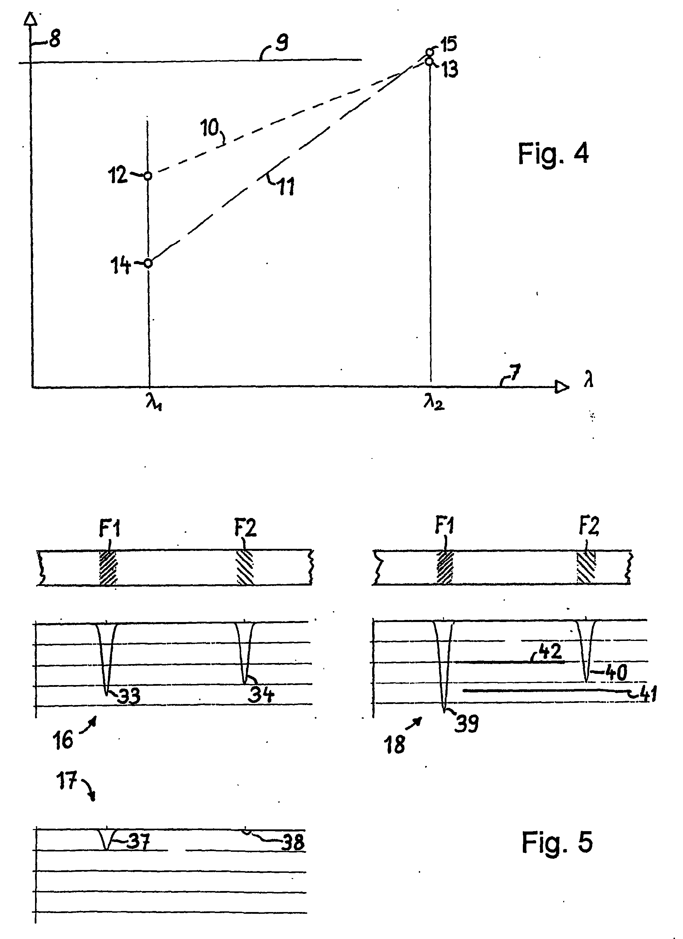

Fig. 4 zeigt ein Diagramm mit einer Achse 7, längs der Werte für die Wellenlänge aufgetragen sind und mit einer Achse 8, längs der Werte für das Ausmass der Reflexion von Strahlen aufgetragen sind. In diesem Diagramm sind verschiedene Linien eingezeichnet, nämlich eine Linie 9 für das Grundmaterial, z.B. Baumwolle, eine kurz unterbrochene Linie 10 für einen ersten Fremdstoff F1 und eine unterbrochene Linie 11 für einen zweiten Fremdstoff F2. Um das Beispiel etwas konkreter einer möglichen realen Aufgabe anzunähern, kann man beispielsweise annehmen, dass als erster Fremdstoff F1 vegetabiles Material, als zweiter Fremdstoff F2 rote Fasern aus Folien verstanden werden könnten. Dies ist lediglich als eine Auswahl möglicher Beispiele für Fremdstoffe aufzufassen. Die Linie 9 entspricht einem Referenzwert für die Reflexion der Strahlen am vorgesehenen Grundmaterial allein. Die Linien 10 und 11 verbinden ausgewählte Punkte 12, 13, 14, 15, die für eine bestimmte Wellenlänge λ₁, λ₂ gemessene Werte der Reflexion darstellen. Die Linien 10 und 11 geben keinen Verlauf der Reflexion in Funktion der Wellenlänge zwischen den Punkten 12 - 15 an, sondern dienen lediglich dazu, die Punkte leichter zu identifizieren, die dem gleichen Fremdstoff entsprechen.

Fig. 5 zeigt schematisch je einen Abschnitt des Grundmaterials oder insbesondere eines Garns mit einem ersten Fremdstoff F1 und einem zweiten Fremdstoff F2. Durch Reflexion erzeugte elektrische Signale 16 und 17 sind für Strahlungen λ₁ und λ₂ angegeben. Ein Signal 18 ergibt sich für gemischte Strahlen, die aus zwei Wellenlängenbereichen λ₁ und λ₂ bestehen. Die horizontalen Abschnitte dieser Signale entsprechen Werten für die Reflexion der Strahlung am reinen Grundmaterial oder am nicht verunreinigten Garn, während nach unten zeigende Ausschläge Werten für die Reflexion entsprechen, wie sie durch Fremdstoffe erzeugt werden können. Auf die Ursachen und die Bedeutung der einzelnen Ausschläge soll im Zusammenhang mit der Beschreibung der Wirkungsweise der Erfindung näher eingegangen werden.

Fig. 6 zeigt eine vereinfachte Darstellung einer Vorrichtung zur Durchführung des erfindungsgemässen Verfahrens. Diese besteht aus einer Strahlungsquelle 19, einem Empfänger 20 für die am Grundmaterial 1, 3, 5 reflektierte Strahlung 44 und einer Auswerteeinheit 21 für die vom Empfänger 20 ausgegebenen elektrischen Signale. Die Strahlungsquelle 19 kann auch mit einer Steuerung 22 verbunden sein. Die Strahlungsquelle 19 besteht beispielsweise aus einer LED als Quelle für Strahlen mit einer ersten Wellenlänge und einer weiteren LED als Quelle für Strahlen mit einer zweiten Wellenlänge. Eine allenfalls dosierte Speisung für die beiden Quellen oder ein Ein- und Ausschalten der einen Quelle erfolgt über die Steuerung 22.

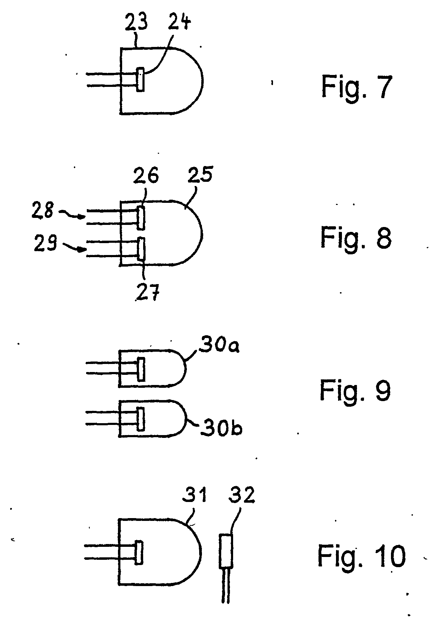

Fig. 7 zeigt als Ausführung für eine Strahlungsquelle 19 eine an sich bekannte Leuchtdiode (bekannterweise als LED bezeichnet) 23 mit einem Chip 24 die Strahlen in einem vorzugsweisen ersten begrenzten Wellenlängenbereich sowie zusätzlich auch Strahlen in einem zweiten begrenzten Wellenlängenbereich wenn auch mit unterschiedlicher Leistung abstrahlen kann.

Fig. 8 zeigt eine Leuchtdiode 25 mit zwei Chips 26 und 27 im selben Gehäuse montiert, wobei jeder Chip 26, 27 mit einer eigenen Wellenlänge strahlt und dafür auch eigene Anschlüsse 28, 29 aufweist.

Fig. 9 zeigt als Ausführung für eine Strahlungsquelle 19 zwei nebeneinander angeordnete Leuchtdioden 30a und 30b, jede für Strahlen in einem eigenen begrenzten Wellenlängenbereich geeignet.

Fig. 10 zeigt als Strahlungsquelle 19 zwei hintereinander geschaltete Leuchtdioden 31 und 32, wobei die Leuchtdode 32 als sogenannte "Chip-LED" Leuchtdiode ausgebildet ist.

### Die Wirkungsweise der Erfindung ist wie folgt:

Geht es beispielsweise darum, in einem Grundmaterial 1, 3, 5 einen ersten Fremdstoff F1 zu erkennen, so wählt man gemäss Fig. 4 einen Wellenlängenbereich λ₁ oder λ₂ aus, in dem der Fremdstoff die ausgesendeten Strahlen genügend stark reflektiert, so dass ein elektrisches Signal entsteht, das sich von demjenigen des Grundmaterials genügend stark unterscheidet. Das wäre für die durch die Linien 10 und 11 dargestellten Fremdstoffe sicher bei λ₁ der Fall. Das dabei entstehende elektrische Signal 16, welches die Reflexion für Strahlen mit λ₁ quantifiziert, ergibt gemäss Fig. 5 für den Fremdstoff F1 einen grossen Ausschlag oder Wert 33 und für den Fremdstoff F2 einen ebenfalls recht grossen Ausschlag oder Wert 34. Das bedeutet, dass in diesem Falle die Fremdstoffe F1 und F2 gut erkennbar sind. Allerdings wird es in der Praxis schwierig aufgrund der geringen Differenz zwischen den Ausschlägen oder Werten 33 und 34 zwischen F1 und F2 zu unterscheiden.

Mit einer Strahlungsquelle 19, die Strahlen 36 mit einer Wellenlänge λ₂ aussendet, zeigt ein Blick auf die Fig. 4, dass es schwierig sein wird, die Fremdstoffe F1 und F2 unter sich und auch gegenüber dem Grundmaterial wie es die Linie 9 charakterisiert zu unterscheiden. Dies bestätigt ein Signalverlauf 17 in Fig. 5, wie er am Ausgang des Empfängers 20 zu erwarten ist mit Ausschlägen oder Werten 37 und 38.

Verwendet man aber eine erfindungsgemässe Strahlungsquelle 19, die Strahlen 35, 36 mit zwei verschiedenen Wellenlängenbereichen aussenden kann, so lässt sich damit ein Signalverlauf 18 am Ausgang des Empfängers 20 erreichen. Strahlen mit den zwei Wellenlängenbereichen λ₁ und λ₂ ergeben für den Fremdstoff F1 einen Ausschlag oder einen Wert 39 und für den Fremdstoff F2 einen Ausschlag oder Wert 40. Man erkennt sofort, dass im Vergleich zum Signalverlauf 16, die Ausschläge 39 und 40 für die Fremdstoffe F1 und F2 sich untereinander stärker unterscheiden als die Ausschläge 33 und 34 des Signalverlaufes 16. Somit ist es hier möglich, zur Diskriminierung der Fremdstoffe F1 und F2 eine Grenze 41 zwischen den maximalen Werten oder . Ausschlägen 39 und 40 zu setzen. Wird die Grenze 41 durch den Signalverlauf 18 überschritten, so werden Fremdstoffe F1 erkannt. Wird zusätzlich eine Grenze 42 unterhalb dem Wert oder Ausschlag 40 vorgegeben, so kann für das Signal 18 festgestellt werden, ob es nur den Fremdstoff F1, nur den Fremdstoff F2 oder beide Fremdstoffe F1 und F2 anzeigt. Mit anderen Worten werden die Fremdstoffe F1 und F2 zusammen erkannt, wenn das Signal 18 die Grenze 42 übertrifft. Es werden Fremdstoffe F1 erkannt, wenn das Signal 18 die Grenze 41 übertrifft.
Auf diese Weise ist es möglich, die Fremdstoffe F1 und F2 für sich gesondert zu erfassen. Dabei basiert der Signalverlauf 18 auf der Annahme, dass beispielsweise 30% der Strahlen die Wellenlänge λ₂ und der Rest die Wellenlänge λ₁ aufweisen.

Mit dem genannten Verfahren ist es somit wie gezeigt möglich, entweder den Fremdstoffe F1 allein oder auch die Fremdstoffe F1 und F2 zusammen zu erkennen. Es ist auch möglich, die relative Intensität der beiden Wellenlängenbereiche wählbar zu machen, um damit beispielsweise Vegetabilien in unterschiedlich wählbarem Masse auszureinigen.

Zur Durchführung des Verfahrens, soll die Vorrichtung vorzugsweise mit einer Strahlungsquelle 19 versehen werden, bei der die Leistung der Strahlen im einen Wellenlängenbereich im Vergleich zum anderen Wellenlängenbereich steuerbar ist. Dies lässt sich mit Strahlungsquellen, wie sie die Figuren 8 bis 10 zeigen, und eine dafür ausgebildete Steuerung 22 leicht erreichen.

Indem man die Steuerung 22 entsprechend gestaltet, kann man auch dafür sorgen, dass die Strahlen im mindestens einen Wellenlängenbereich ein- und ausschaltbar sind. So kann das Bedienungspersonal die Anlage auch leicht auf ein neues Grundmaterial oder auf andere Fremdstoffe einstellen.

Es kann bei der Verarbeitung von Baumwolle zu Garn wichtig sein, die Zahl der Eingriffe eines an sich bekannten Garnreinigers zu begrenzen. Diese Eingriffe geschehen bei der Produktion von Garn mit an sich bekannten Gamreinigem insbesondere mit deren Schneidelement. Allerdings ist zu bedenken, dass je mehr Fremdstoffe erkannt und ausgeschieden werden, desto häufiger die Produktionsmaschine, also die Spinnmaschine oder Spulmaschine stillgesetzt wird. Deshalb ist es wichtig zu entscheiden welche Fremdstoffe toleriert werden oder welche herausgeschnitten werden sollen. Das kann beispielsweise dadurch geschehen, dass man vorgehend darüber entscheidet, welche Fremdstoffe im Endprodukt wirklich schädlich sind und welche nicht. Beispielsweise sind sogenannte Vegetabilien als Fremdstoffe zwar unerwünscht, aber auch recht harmlos, da sie beispielsweise die Färbbarkeit des Garns nicht beeinträchtigen und sich somit im Gewebe nicht leicht erkennen lassen. Strahlen in einem Wellenlängebereich zwischen 520 und 570 nm eignen sich gut um andere Fremdstoffe wie z.B. grüne, rote oder blaue Polypropylenfolie als Fremdstoffe zu erkennen und die Vegetabilien zu diskriminieren. In Bezug auf die Ausführungen zu Fig. 4 und 5 würde dann die Polyproylenfolie etwa dem Fremdstoff F2, die Vegetabilien dagegen dem Fremdstoff F1 etwa entsprechen.

Für den zweiten Wellenlängenbereich λ₂ eignen sich beispielsweise Strahlen mit einer Wellenlänge im Infrarotbereich.

Ist ein Gerät 43 zum Ausscheiden der Fremdstoffe aus dem Grundmaterial, wie z.B. ein an sich bekannter Garnreiniger vorgesehen, so kann dieses durch die Auswerteeinheit 21 aktiviert werden, sobald die darin beispielsweise in einem Prozessor vorgespeicherten Grenzen 41, 42 erreicht oder überschritten werden. Die Grenzen können über ein Eingabegerät in die Auswerteeinheit 21 eingegeben werden.

Bei der Produktion von Garn, wie in diesem Beispiel angenommen, kann auf diese Weise die Produktion dahingehend optimiert werden, dass klar unterschieden werden kann, welche Fremdstoffe derart störend sind, dass man es erlaubt, dass diese entfernt werden, was immer auch bedeutet, dass für die Zeit, die man zur Entfernung braucht, die Produktion, d.h. die Spinnstelle oder die Spulstelle stillsteht. Wenn man also vor der Produktion abklärt welche Fremdstoffe wahrscheinlich im Grundmaterial vorhanden sind und welche man wirklich entfernen will, so kann damit einerseits die Qualität des Produktes gesteigert und andererseits die Leistung der Produktion erhalten werden indem beispielsweise die Schnittzahl des Garnreinigers beschränkt wird. Sollen Fremdstoffe statt wie gezeigt in einem Garn, in einem Vorgarn, einem Band oder in einem Flächengewebe erkannt werden, so hat das lediglich einen Einfluss auf die Dimensionierung der Strahlungsquellen und der Empfänger, insbesondere aber auf das Gerät zum Ausschneiden der Fremdstoffe, sofern ein solches vorhanden ist.

## Patentansprüche

1. Verfahren zur Erkennung von Fremdstoffen in einem textilen Grundmaterial (1, 3, 5), bei dem
das textile Grundmaterial mit Strahlen (35, 36), die im Wesentlichen zwei begrenzte und unterschiedliche Wellenlängenbereiche (λ₁ und λ₂) aufweisen, beaufschlagt wird und
die am Grundmaterial reflektierten Strahlen (44) aus beiden Wellenlängenbereichen gleichzeitig und gemeinsam erfasst und in ein elektrisches Signal (18) gewandelt werden,
**dadurch gekennzeichnet,**
**dass** die beiden Wellenlängenbereiche (λ₁ und λ₂) derart gewählt werden, dass für die ausgesendeten Strahlen (35, 36) hinsichtlich der Intensität ihrer Reflexion an zwei gesuchten Fremdstoffen (F1, F2) genügende Unterschiede erwartet werden, und
**dass** die beiden Fremdstoffe (F1, F2) durch eine Grenze (41), die zwischen Ausschlägen (39, 40) des elektrischen Signals (18) für die beiden Fremdstoffe (F1, F2) liegt, voneinander unterschieden werden.

2. Verfahren nach Anspruch 1, **dadurch gekennzeichnet,**
**dass** ein erster begrenzter Wellenlängenbereich (λ₁) der Strahlen so gewählt wird, dass die am Grundmaterial mit Fremdstoffen reflektierten Strahlen für mindestens zwei verschiedene Fremdstoffe (F1, F2) im elektrischen Signal erste Werte (33, 34) ergeben,
**dass** ein zweiter begrenzter Wellenlängenbereich (λ₂) so gewählt wird, dass die reflektierten Strahlen für die selben Fremdstoffe (F1, F2) ein elektrisches Signal mit zweiten Werten (37, 38) ergeben, welche für die beiden Fremdstoffe nicht im gleichen Verhältnis stehen wie bei den entsprechenden elektrischen Signalen im ersten Wellenlängenbereich.

3. Verfahren nach Anspruch 1, **dadurch gekennzeichnet, dass** die Leistung der Strahlen im einen Wellenlängenbereiche im Vergleich zum anderen Wellenlängenbereich steuerbar ist.

4. Verfahren nach Anspruch 1, **dadurch gekennzeichnet, dass** die Strahlen im mindestens einen Wellenlängenbereich ein- und ausschaltbar sind.

5. Verfahren nach Anspruch 1, **dadurch gekennzeichnet, dass** für einen Wellenlängenbereich Strahlen mit einer Wellenlänge im infrarotbereich gewählt werden.

6. Verfahren nach Anspruch 1, **dadurch gekennzeichnet, dass** der eine Wellenlängenbereich zur Unterscheidung von störenden Fremdstoffen in einem Garn als textilem Grundmaterial vorgesehen wird.

## Claims

1. A method for recognizing foreign matter in a textile base material (1, 3, 5), with which the textile base material is subjected to beams (35, 36) which have essentially two limited and different wavelength regions (λ₁ and λ₂) and
the beams (44) of both wavelength regions which are reflected at the base material are detected simultaneously and together and are converted into an electrical signal,
**characterised in that**
the two wavelength regions (λ₁ and λ₂) are selected in a manner such that sufficient differences are expected for the emitted beams (35, 36), with regard to the intensity of their reflection at two sought foreign matters (F1, F2), and
that the two foreign matters (F1, F2) are differentiated from one another by way of a border (41) which lies between swings (39, 40) of the electrical signal (18) for the two foreign matters (F1, F2).

2. The method according to claim 1, **characterised in that**
a first limited wavelength region (λ₁) of the beams is selected such that the beams reflected at the base material with foreign manner yield first values (33, 34) in the electrical signal for at least two different foreign matters (F1, F2),
that a second limited wavelength region (λ₂) is selected such that the reflected beams for the same foreign matters (F1, F2) yield an electrical signal with two values (37, 38) which for the two foreign matters do not have the same ratio as with the respective electrical signals in the first wavelength region.

3. A method according to claim 1, **characterised in that** the power of the beams in the one wavelength region is controllable in comparison to the other wavelength region.

4. A method according to claim 1, **characterised in that** the beam are switchable on and off in at least one wavelength region.

5. A method according to claim 1, **characterised in that** beams with a wavelength in the infrared region are selected for one wavelength region.

6. A method according to claim 1, **characterised in that** the one wavelength region is provided for the differentiation of disturbing foreign matter in a yarn as a textile base material.

## Revendications

1. Procédé pour identifier des corps étrangers dans un matériau de base textile (1, 3, 5), dans lequel le matériau de base textile est chargé par des rayons (35, 36) qui présentent essentiellement deux zones de longueur d'onde limitées et différentes (λ₁ et λ₂), et
les rayons (44) réfléchis au matériau de base des deux zones de longueur d'onde sont détectées en même temps et conjointement et sont convertis en un signal électrique (18),
**caractérisé**
**en ce que** les deux zones de longueur d'onde (λ₁ et λ₂) sont sélectionnées de telle sorte que pour les rayons émis (35, 36), concernant l'intensité de leur réflexion à deux corps étrangers recherchés (F1, F2), des différences suffisantes sont attendues, et
**en ce que** les deux corps étrangers (F1, F2) sont distingués l'un de l'autre par une limite (41) qui se situe entre des amplitues (39, 40) du signal électrique (18) pour les deux corps étrangers (F1, F2).

2. Procédé selon la revendication 1, **caractérisé en ce qu'**une première zone de longueur d'onde limitée ((λ₁) des rayons est sélectionnée de façon que les rayons réfléchis au matériau de base avec des corps étrangers, pour au moins deux corps étrangers différents (F1, F2), fournissent dans le signal électrique des premières valeurs (33, 34)
**en ce qu'**une deuxième zone de longueur d'onde limitée (λ₂) est sélectionnée de façon que les rayons réfléchis pour les mêmes corps étrangers (F1, F2) fournissent un signal électrique avec des deuxièmes valeurs (37, 38) qui, pour les deux corps étrangers, ne sont pas dans le même rapport que dans les signaux électriques correspondants dans la première zone de longueur d'onde.

3. Procédé selon la revendication 1, **caractérisé en ce que** la puissance des rayons peut être commandée dans une plage de longueur d'onde en comparaison avec l'autre plage de longueur d'onde.

4. Procédé selon la revendication 1, **caractérisé en ce que** les rayons dans au moins une plage de longueur d'onde peuvent être mis en et hors service.

5. Procédé selon la revendication 1, **caractérisé en ce que** pour une zone de longueur d'onde, des rayons d'une longueur d'onde dans la plage infrarouge sont sélectionnés.

6. Procédé selon la revendication 1, **caractérisé en ce qu'**une plage de longueur d'onde est prévue pour différencier des corps étrangers gênants dans un fil comme matériau de base textile.
